Europäisches Patentamt

European Patent Office

Office européen des brevets

(19)

(11) Veröffentlichungsnummer : **0 360 095 B1**

(12) ## EUROPÄISCHE PATENTSCHRIFT

(45) Veröffentlichungstag der Patentschrift :
**15.04.92 Patentblatt 92/16**

(51) Int. Cl.$^5$ : **C07C 65/05, C07C 51/15**

(21) Anmeldenummer : **89116626.6**

(22) Anmeldetag : **08.09.89**

(54) **Verfahren zur Herstellung von 2,4-Dihydroxybenzoesäure.**

(30) Priorität : **21.09.88 DE 3832076**

(43) Veröffentlichungstag der Anmeldung :
**28.03.90 Patentblatt 90/13**

(45) Bekanntmachung des Hinweises auf die
Patenterteilung :
**15.04.92 Patentblatt 92/16**

(84) Benannte Vertragsstaaten :
**CH DE FR GB IT LI**

(56) Entgegenhaltungen :
**GB-A- 734 598**
**GB-A- 734 622**

(73) Patentinhaber : **BASF Aktiengesellschaft**
**Carl-Bosch-Strasse 38**
**W-6700 Ludwigshafen (DE)**

(72) Erfinder : **Die Erfinder haben auf ihre Nennung**
**verzichtet**

## Beschreibung

Die vorliegende Erfindung betrifft ein neues Verfahren zur Herstellung von 2,4-Dihydroxybenzoesäure in einem Feststoffmischer durch Umsetzung von Resorcin mit Alkalihydrogencarbonat und/oder Alkalicarbonat in einer Kohlendioxid-Atmosphäre, wobei man als Alkalihydrogencarbonat oder Alkalicarbonat jeweils eine Mischung der entsprechenden Natrium- und Kaliumsalze verwendet.

Die Herstellung von Benzoesäuren durch Carboxylierung im alkalischen Milieu ist unter dem Namen Kolbe-Synthese allgemein bekannt. Bei der Verwendung von Resorcin als Aromat ist neben der Umsetzung in Lösungsmitteln, bei der im allgemeinen eine schlechte Ausbeute und eine geringe Selektivität zu erwarten ist, auch die Festphasencarboxylierung nach Marassé bekannt, die mit Kohlendioxid in Gegenwart von Kaliumcarbonat durchgeführt wird (J. Org. Chem. 19, 510 (1954); Monatsh. 81, 1071 (1950); J. Org. Chem. 15, 233 (1950)).

Es gilt die Lehre, daß die Marassé-Reaktion nur unter Verwendung der Kalium-, Rubidium- oder Caesiumsalze erfolgreich durchgeführt werden kann. Dagegen gelten die billigeren Natriumsalze als ungeeignet (Chem. Rev. 57, 583 (1957)).

Aufgabe der vorliegenden Erfindung war es nun, ein Verfahren zur Herstellung von 2,4-Dihydroxybenzoesäure mittels Festphasencarboxylierung bereitzustellen, bei dem auch Natriumhydrogencarbonat und/oder Natriumcarbonat mit Erfolg angewendet werden können.

Es wurde gefunden, daß die Herstellung von 2,4-Dihydroxybenzoesäure in einem Feststoffmischer durch Umsetzung von Resorcin mit Alkalihydrogencarbonat, Alkalicarbonat oder deren Mischung in einer Kohlendioxid-Atmosphäre vorteilhaft gelingt, wenn man als Alkalihydrogencarbonat oder Alkalicarbonat jeweils eine Mischung der entsprechenden Natrium- und Kaliumsalze verwendet.

Der Anteil der Kaliumsalze in der Mischung beträgt hierbei in der Regel 10 bis 60 Gew.-%, vorzugsweise 20 bis 40 Gew.-%, jeweils bezogen auf das Gewicht der Summe aus Natrium- und Kaliumsalz.

Bevorzugt ist die Verwendung einer Mischung aus Natrium- und Kaliumhydrogencarbonat.

Im allgemeinen werden pro Mol Resorcin 400 bis 1500 g, vorzugsweise 450 bis 700 g und insbesondere 500 bis 600 g des Gemisches aus Natrium- und Kaliumhydrogencarbonat und/oder -carbonat verwendet.

Das erfindungsgemäße Verfahren wird zweckmäßig bei einer Temperatur von 80 bis 140°C, vorzugsweise 100 bis 120°C durchgeführt.

Das bei der Umsetzung benötigte Kohlendioxid wird vorzugsweise bei atmosphärischem Druck der Reaktion zugeführt. Es ist aber auch möglich unter erhöhtem Druck, im allgemeinen bis zu ca. 20 bar, zu arbeiten. Das Kohlendioxid kann auch durch Inertgase, z.B. Stickstoff, bis zu einem Partialdruck von 0,5 bar verdünnt werden.

Dem Reaktionsgemisch können weiterhin Inertstoffe, beispielsweise anorganische Salze, z.B. Halogenide, Sulfate oder Phosphate von Alkalimetallen oder Erdalkalimetallen oder auch Erdalkalihydrogencarbonate oder Erdalkalicarbonate, zugesetzt werden, ohne daß die Umsetzung dabei beeinträchtigt wird. Weiterhin können auch inerte Werkstoffe, wie Mineralien oder Metalle zugesetzt werden. Im allgemeinen beträgt die Menge an zugesetzten Inertstoffen 0 bis 200 Gew.-%, vorzugsweise 0 bis 100 Gew.-%, jeweils bezogen auf das Reaktionsgemisch.

Das erfindungsgemäße Verfahren wird in einem Feststoffmischer durchgeführt. Solche Appraturen sind bekannt und z.B. in Ullmanns Encyklopädie der Technischen Chemie, 4. Auflage, Band 2, 5. 304 bis 310, beschrieben.

Man führt das neue Verfahren zweckmäßig so durch, daß man Resorcin und das Gemisch aus Natrium- und Kaliumhydrogencarbonat und/oder -carbonat, gegebenenfalls unter Zusatz der obengenannten Inertstoffe, im Feststoffmischer vorlegt und unter Mischbedingungen Kohlendioxid einströmen läßt. Nach Erhitzen auf die obengenannte Temperatur und einer Reaktionsdauer von ca. 1 bis 10 Stunden, vorzugsweise ca. 2 bis 4 Stunden, unter den oben aufgeführten Reaktionsbedingungen ist die Umsetzung beendet.

Zur Aufarbeitung kann das feste Reaktionsgemisch in Wasser eingetragen und das resultierende Gemisch daraufhin angesäuert werden. Die dabei sich abscheidende 2,4-Dihydroxybenzoesäure kann dann abgetrennt und getrocknet werden.

Es ist auch möglich, das feste Reaktionsgemisch mit niederen Alkoholen, wie Methanol, Ethanol oder Isopropanol, zu extrahieren, wobei die Extraktionstemperatur zwischen Raumtemperatur und dem Siedepunkt des jeweils verwendeten Extraktionsmittels liegen kann. Nach Entfernen des Extraktionsmittels erhält man ein Gemisch aus dem Natrium- und Kaliumsalz der 2,4-Dihydroxybenzoesäure, das entweder als solches verwendet oder durch Ansäuern in die freie Säure umgewandelt werden kann. Der Extraktionsrückstand besteht weitestgehend aus Natrium- und Kaliumhydrogencarbonat und/oder -carbonat und kann nach dem Trocknen erneut in die Festphasenreaktion eingesetzt werden.

Das erfindungsgemäße Verfahren kann sowohl in kontinuierlicher als auch diskontinuierlicher Arbeitsweise durchgeführt werden.

Die Mischung der Natrium- und Kaliumsalze der 2,4-Dihydroxybenzoesäure kann mittels niederen Alkoholen aus dem Reaktionsgemisch extrahiert werden.

2,4-Dihydroxybenzoesäure ist ein wertvolles Zwischenprodukt für die Herstellung von UV-Absorbern.

Die folgenden Beispiele sollen die Erfindung näher erläutern.

Beispiel 1

In einem 7-l-Feststoffmischer wurden 1 kg Kaliumhydrogencarbonat, 2 kg Natriumhydrogencarbonat und 700 g Resorcin vorgelegt und unter Mischbedingungen in einem Strom von Kohlendioxid auf 110°C erwärmt. Nach 3 Stunden ließ man unter Mischbedingungen abkühlen, trug das pulverförmige Produkt (2,77 kg) in 10 l Wasser ein und stellte mit konz. Salzsäure auf pH 3. Nach Absaugen und Trocknen erhielt man 589 g (60 %) 2,4-Dihydroxybenzoesäure; Reinheit (HPLC): 99,1 %.

Beispiel 2

In einem 7-l-Feststoffmischer wurden 1 kg Kaliumhydrogencarbonat, 2 kg Natriumhydrogencarbonat und 550 g Resorcin vorgelegt und unter Mischbedingungen in einem Strom von Kohlendioxid auf 110°C erwärmt. Nach 3 Stunden ließ man unter Mischbedingungen abkühlen, trug das pulverförmige Produkt (2,66 kg) in 6,7 l Wasser ein und stellte mit konz. Salzsäure auf pH 3. Nach Absaugen und Trocknen erhielt man 563 g (73 %) 2,4-Dihydroxybenzoesäure; Reinheit (HPLC): 99,3 %.

Beispiel 3

In einem 7-l-Feststoffmischer wurden 1,5 kg Kaliumhydrogencarbonat, 1,5 kg Natriumhydrogencarbonat und 550 g Resorcin vorgelegt und unter Mischbedingungen in einem Strom von Kohlendioxid auf 110°C erwärmt. Nach 3 Stunden ließ man unter Mischbedingungen abkühlen, trug das pulverförmige Produkt (2,93 kg) in 10 l Wasser ein und stellte mit konz. Salzsäure auf pH 3. Nach Absaugen und Trocknen erhielt man 575 g (75 %) 2,4-Dihydroxybenzoesäure; Reinheit (HPLC): 98,3 %.

Beispiel 4

In einem 7-l-Feststoffmischer wurden 1 kg Kaliumhydrogencarbonat, 2 kg Natriumhydrogencarbonat und 550 g Resorcin vorgelegt und unter Mischbedingungen in einem Strom von Kohlendioxid auf 110°C erwärmt. Nach 3 Stunden ließ man unter Mischbedingungen abkühlen, trug das pulverförmige Produkt in 4 l Methanol ein und erhitzte unter Rühren 1 Stunde unter Rückfluß. Der Rückstand wurde abgesaugt und getrocknet (1,25 kg). Das Filtrat wurde eingeengt, der resultierende Rückstand mit Wasser aufgenommen und auf pH 3 gestellt. Nach Absaugen und Trocknen erhielt man 216 g 2,4-Dihyroxybenzoesäure, Reinheit (HPLC): 99,7 %.

Beispiel 5

In einem 35-l-Feststoffmischer wurden 10 kg Natriumhydrogencarbonat, 5 kg Kaliumhydrogencarbonat und 3,3 kg Resorcin vorgelegt und unter Mischbedingungen in einem Strom von Kohlendioxid auf 120°C erwärmt. Nach 3 Stunden ließ man unter Mischbedingungen abkühlen und gab 28 l Wasser zu. Die auf 60°C erwärmte Suspension wurde dann abgelassen und bei Raumtemperatur mit 20 kg konz. Salzsäure auf pH 3 gestellt. Die Suspension wurde auf 10 bis 15°C abgekühlt, mit einer Filterpresse abfiltriert, mit Wasser gewaschen und getrocknet. Man erhielt 4,1 kg (81 %) 2,4-Dihydroxybenzoesäure; Reinheit (HPLC): 99,5 %.

**Patentansprüche**

1. Verfahren zur Herstellung von 2,4-Dihydroxybenzoesäure in einem Feststoffmischer durch Umsetzung von Resorcin mit Alkalihydrogencarbonat, Alkalicarbonat oder deren Mischung in einer Kohlendioxid-Atmosphäre, dadurch gekennzeichnet, daß man als Alkalihydrogencarbonat oder Alkalicarbonat jeweils eine Mischung der entsprechenden Natrium-und Kaliumsalze verwendet.

2. Verfahren gemäß Anspruch 1, dadurch gekennzeichnet, daß man eine Mischung aus Natrium- und Kaliumhydrogencarbonat verwendet.

3. Verfahren gemäß Anspruch 1, dadurch gekennzeichnet, daß man die Umsetzung bei einer Temperatur von 80 bis 140°C vornimmt.

4. Verfahren gemäß Anspruch 1, dadurch gekennzeichnet, daß man die Umsetzung bei atmosphärischem Druck vornimmt.

**Claims**

1. A process for preparing 2,4-dihydroxybenzoic acid in a solids mixer by reacting resorcinol with an alkali metal bicarbonate, an alkali metal carbonate or a mixture thereof in a carbon dioxide atmosphere, which comprises using as the alkali metal bicarbonate or alkali metal carbonate a mixture of the corresponding sodium and potassium salts.

2. A process as claimed in claim 1, wherein a mixture of sodium bicarbonate and potassium bicarbonate is used.

3. A process as claimed in claim 1, wherein the reaction is carried out at from 80 to 140°C.

4. A process as claimed in claim 1, wherein the reaction is carried out under atmospheric pressure.

**Revendications**

1. Procédé de préparation d'acide 2,4-dihydroxybenzoïque dans un mélangeur pour matières solides, par réaction de résorcine avec un hydrogénocarbonate alcalin, un carbonate alcalin ou un mélange de ceux-ci dans une atmosphère d'anhydride carbonique, caractérisé en ce qu'on utilise, comme hydrogénocarbonate alcalin ou carbonate alcalin, un mélange des sels de sodium et de potassium correspondants.

2. Procédé selon la revendication 1, caractérisé en ce qu'on utilise un mélange d'hydrogénocarbonates de sodium et de potassium.

3. Procédé selon la revendication 1, caractérisé en ce qu'on mène la réaction à une température de 80 à 140°C.

4. Procédé selon la revendication 1, caractérisé en ce qu'on mène la réaction à la pression atmosphérique.